# EUROPEAN PATENT SPECIFICATION

(11) **EP 2 871 181 B1**
(45) Date of publication and mention of the grant of the patent: **05.10.2016**
(21) Application number: 13192475.5
(22) Date of filing: 12.11.2013
(51) Int. Cl.: C07D 263/34, A61K 31/421, A61P 27/16

(54) **Novel Compounds for Regeneration of Terminally-Differentiated Cells and tissues**
Neue Verbindungen zur Regenerierung von terminal-differenzierten Zellen und Geweben
Nouveaux composés pour la régénération de cellules et de tissus terminalement différenciés

(43) Date of publication of application: 13.05.2015
(73) Proprietor: Acousia Therapeutics GmbH, 72070 Tübingen (DE)
(72) Inventor: Dr. Bös, Michael, 72070 Tübingen (DE)
(74) Representative: Patentanwälte Ruff, Wilhelm, Beier, Dauster & Partner mbB

(56) References cited:
- WO-A1-2011/095338
- WO-A2-2007/133865
- WIPF P ET AL: "A New Synthesis of alpha-Methylserine by Nucleophilic Ring-Opening of N-Sulfonyl Aziridines", TETRAHEDRON LETTERS, PERGAMON, GB, vol. 36, no. 21, 22 May 1995 (1995-05-22), pages 3639-3642, XP004028039, ISSN: 0040-4039, DOI: 10.1016/0040-4039(95)00621-I

## Description

The present invention relates to novel compounds in the nature of oxazole carboxylic acid amides and thiazole carboxylic acid amides which are useful in the regeneration of post-mitotic cells and tissues. The present invention also relates to medicaments and pharmaceutical compositions comprising these compounds.

In mammals there is only a limited number of organs and tissues which have the ability for regeneration after damage. Organs and tissues with such an (normally limited) ability are liver, bones or skin.

Many other mammalian organs and tissues, e.g. heart, brain, skeleton muscle are not able to regenerate after damage. The reason is that the corresponding cells in such organs and tissues leave the cell cycle irreversibly and remain in a terminally differentiated state. Therefore, e.g. a myocardial infarction (heart) or a stroke (brain) lead to an irreversible damage of the affected tissues.

Further, the sensory epithelia of the eye and the inner ear of mammals do not have the ability for regeneration after damage. In the case of the inner ear such damage often results e.g. in hardness of hearing in humans which is clearly associated with a reduction in quality of life.

In this context, it is estimated that approximately 10% of the population of the industrialized nations is affected by hardness of hearing. The vast majority of these cases can be attributed to a so-called sensorineural hearing loss which is characterized initially by a high frequency hearing loss affecting the ability to hear and understand speech. This sensorineural hearing loss or sensorineural deafness results mainly from damage to cells in the inner ear known as "hair" cells. These highly complex sensory cells detect the sound vibrations which are passed from outside, via the ear drum and the bones of the middle ear, to the cochlea. These sensory hair cells are located in the so-called organ of Corti.

The most frequent reasons for a loss of sensory hair cells are age-related degeneration, exposure to noise, side-effects of (ototoxic) medicaments, genetic defects and others.

As, surprisingly, it was discovered that, after acoustic trauma and ototoxic damage, avian cochlea is capable to regenerate sensory hair cells spontaneously, there were attempts to apply this discovery also to mammals, in particular humans. This was done on the basis of the biological mechanism for sensory hair cell regeneration in avian cochlea that supportive cells directly adjacent to the destroyed sensory hair cells undergo cell division resulting in a population of undifferentiated cells which are capable of redifferentiation to newly formed sensory hair cells and supportive cells.

However, all these attempts with respect to a regeneration of sensory hair cells in mammals were not successful, at least not up to now.

As a consequence, at the time being sensorineural hearing loss can (only) be treated with hearing aids, which amplify sounds at preset frequencies to overcome a sensorineural hearing loss in that range. It can also be treated with cochlea implants which stimulate cochlea nerves directly.

Nevertheless, there are still efforts made to identify compounds, in particular low-molecular weight compounds which can stimulate an endogenous regeneration of terminally differentiated cells and tissues in mammals. A group of such compounds is e.g. disclosed in WO-A1 2011/095338.

Therefore, it is an object of the present invention to provide a novel group of compounds which are capable to stimulate endogenous regeneration of post-mitotic cells and tissues, in particular in mammals.

The present invention provides novel compounds in the nature of oxazole carboxylic acid amides and thiazole carboxylic acid amides represented by the general formula (I): 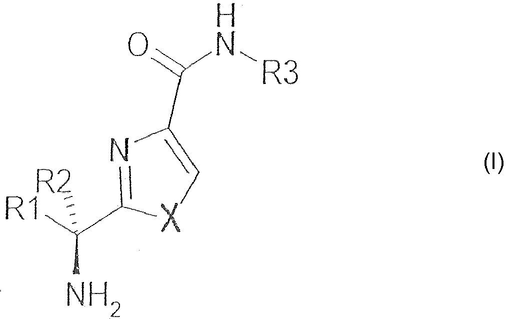 wherein
- X is O (oxygen) or S (sulphur),
- R1 is a substituent selected from the group consisting of straight-chain (unbranched) or branched, unsubstituted or substituted aryl groups, alkylaryl groups, and arylalkyl groups, which optionally contain heteroatoms,
- R2 is a substituent selected from the group consisting of straight-chain (unbranched) or branched, unsubstituted or substituted C1 - C6 alkyl groups, C1 - C6 alkoxy groups, C1 - C6 alkoxyalkyl groups and C2 - C6 alkenyl groups,
- R3 is is a substituent selected from the group consisting of straight-chain (unbranched) or branched, unsubstituted or substituted alkyl groups, cycloalkyl groups, alkylcycloalkyl groups, aryl groups, alkylaryl groups, arylalkyl groups, cycloalkylaryl groups and arylcycloalkyl groups, which optionally contain heteroatoms,
- or a stereoisomer, a tautomer or a pharmaceutically acceptable salt thereof.

Compounds of formula (I) wherein X is O are preferred.

Referring to substituent R1 it is further preferred according to the invention, if R1 is a substituent selected from the group consisting of straight-chain or branched, unsubstituted or substituted heteroaryl groups, alkylheteroaryl groups, and heteroarylalkyl groups. In particular, in these cases R1 is a substituent selected from indolyl groups, alkylindolyl groups, and indolylalkyl groups.

According to the present invention also preferred are compounds, wherein R2 is a substituent selected from the group consisting of straight-chain or branched, unsubstituted or substituted C1 - C6 alkyl groups. In particular, these alkyl groups are C1 - C3 alkyl groups, wherein methyl groups are further preferred.

According to the invention compounds are also preferred, wherein R3 is a substituent selected from the group consisting of straight-chain or branched, unsubstituted or substituted alkyl groups, cycloalkyl groups, and alkylcycloalkyl groups. In particular R3 is a substituent selected from the group consisting of unsubstituted or substituted cycloalkyl groups.

In accordance with the above disclosure the following compounds are preferred with the present invention, namely compounds wherein
- X is O (oxygen),
- R1 is a substituent selected from the group consisting of straight-chain or branched, unsubstituted or substituted indolyl groups, alkylindolyl groups and indolylalkyl groups,
- R2 is a substituent selected from the group consisting of straight-chain or branched, unsubstituted or substituted C1 - C3 alkyl, in particular methyl,
- R3 is a substituent selected from the group consisting of unsubstituted or substituted cycloalkyl groups, in particular cyclohexyl groups.

A most preferred compound according to the invention is the compound, wherein
- X is O (oxygen),
- R1 is (1 H-indol-3-yl)-methyl,
- R2 is methyl,
- R3 is cyclohexyl.

All compounds according to the invention as mentioned above can be used as or in a medicament or pharmaceutical composition.

The use of the inventive compounds for the therapy of a disorder associated with damaged post-mitotic tissues in mammals is preferred. In particular, said tissues are tissues of the inner ear of mammals, wherein the disorder to be treated is an inner ear hearing loss after damage or loss of sensory hair cells in an organ of Corti.

As a consequence, the invention further provides a pharmaceutical composition or medicament comprising:
- at least one inventive compound as claimed and defined above, and
- a pharmaceutically acceptable carrier or diluent.

Finally, a method for treating a disorder in a mammal in need of such treatment is disclosed, wherein
- the disorder comprises a disorder associated with damaged post-mitotic tissues, in particular an inner ear hearing loss after damage or loss of sensory hair cells in an organ of Corti, and
- the method comprises administering to said mammal a therapeutically effective amount of the compound as claimed and as defined above.

In particular, the above-mentioned mammal is a human.

The terms used in the claims and in the above description are defined as follows.

The term "straight chain" as used herein, means a chemical structure in the form of an unbranched chain of atoms in a molecule with no attached side chains. Preferably said (unbranched) chain is an open chain. In contrast to that a "branched" structure includes one or more side chains attached to a chain of atoms in a molecule.

The term "substituted", as used herein, means that anyone or more hydrogens in the corresponding groups is replaced by another atom or group. E.g. "substituted alkyl" refers to an alkyl group in which one or more hydrogens are substituted, e.g. by halogen, hydroxy, or other atoms or groups. "Halogen" refers to fluorine, chlorine, bromine and iodine.

The term "alkyl" refers to (straight or branched chain) hydrocarbon groups of 1 to 20 carbon atoms, preferably 1 to 6 carbon atoms. In general, herein the terms C1, C2, C6, C20 and the like refer to the number of C-atoms (carbon atoms) present in the corresponding groups. Example alkyl groups include, but are not limited to, methyl, ethyl, propyl (e.g., n-propyl and isopropyl), butyl (e.g., n-butyl, isobutyl, t-butyl), and pentyl (e.g., n-pentyl, isopentyl, neopentyl).

The term "cycloalkyl" refers to a saturated cyclic hydrocarbon ring system. Exemplary groups include cyclopropyl, cyclobutyl, cyclopentyl, cyclohexyl, cycloheptyl, cyclooctyl, cyclodecyl, adamantyl and others.

The term "alkylcycloalkyl" refers to an alkyl bonded to a cycloalkyl.

The term "aryl" refers to any functional group or substituent derived from an aromatic ring. Aryl includes, but is not limited to, phenyl, 1-naphthyl, 2-naphthyl, thienyl, indolyl and others. As a consequence, the term "heteroaryl" refers to any group or substituent derived from a heteroaromatic ring. E.g. indolyl is derived from indole, which is a bicyclic heteroaromate, consisting of a six-membered benzene ring fused to a five-membered nitrogen-containing pyrrole ring.

The terms "alkylaryl", "arylalkyl", "cycloalkylaryl", and "arylcycloalkyl" refer to an alkyl bonded to an aryl, an aryl bonded to an alkyl, a cycloalkyl bonded to an aryl and an aryl bonded to a cycloalkyl, resp..

The term "heteroatom" shall include oxygen, sulphur and nitrogen.

The term "alkoxy" refers to an alkyl group bonded to oxygen. Alkoxy includes, but is not limited to, methoxy, ethoxy and others. The term "alkoxyalkyl" refers to an alkoxy group having (another) alkyl group bonded to the oxygen of the alkoxy group. Alkoxyalkyl includes, but is not limited to, methoxymethyl, ethoxyethyl and others.

The term "alkenyl" refers to hydrocarbon groups, having at least one double bond.

The definition of compounds according to the invention includes all possible "stereoisomers" and their mixtures. In particular, the racemic forms and the isolated optical isomers having the specified activity are included. The racemic forms can be resolved by physical methods, such as, for example fractional crystallisation, separation or crystallisation of diastereomeric derivatives or separation by chiral column chromatography. The individual optical isomers can be obtained from the racemates from the conventional methods, such as, for example, salt formation with an optically active acid followed by crystallisation.

The term "tautomers" refers to constitutional isomers of the inventive compounds that readily interconvert by a chemical reaction called tautomerisation. This reaction commonly results in the formal migration of a hydrogen atom or proton, accompanied by a switch of a single bond and adjacent double bond.

The inventive compounds of formula (I) may also have "prodrug" forms. Since prodrugs are known to enhance qualities of pharmaceuticals (e.g., solubility, manufacturing etc.) the compounds of the present invention may be delivered in prodrug form. "Prodrugs" are intended to include any covalently bonded carriers that release an active parent drug of the present invention in vivo when such prodrug is administered to a mammalian subject. Prodrugs include compounds of the present invention wherein e.g. a hydroxyl, amino or other group is bonded to any group that, when the prodrug is administered, cleaves to form a free hydroxyl, free amino or other, resp.. Examples of prodrugs include, but are not limited to, acetate, formate, and benzoate derivates of alcohol and amine function groups in the compounds of the present invention. Various forms of prodrugs are well-known in the art. In this context, according to the invention, prodrug esters or prodrug peptides can be used as prodrug compounds. In certain cases, by coupling cell penetration-enhancing molecules such as, for example, biotin or maleimidopropionic acid, optionally via suitable spacer molecules, to the primary amino group, or by acylation of this amino group, it is possible to improve the bioavailability and thus the efficacy of the compounds according to the invention.

The phrase "pharmaceutically acceptable salts" refers to derivates of the disclosed compounds wherein the parent compound is modified by making acid or base salts thereof. Examples include, but are not limited to, mineral or organic acid salts of basic groups such as amines; and alkali and organic salts of acidic groups such as carboxylic acids. The pharmaceutically acceptable salts include the conventional non-toxic salts or the quaternary ammonium salts formed, for example, from non-toxic inorganic or organic acids. For example, such conventional non-toxic salts include those derived from inorganic acids such as hydrochloric, hydrobromic, sulphuric, phosphoric, and nitric; and the salts prepared from organic acids such as acetic, propionic and others.

The phrase "pharmaceutically acceptable carrier" and the phrase "pharmaceutically acceptable diluent" refer to media generally accepted in the art for the delivery of biologically active agents to animals, in particular mammals. Such media are well-known in the art.

The phrase "therapeutically effective amount" is intended to include an amount of a compound according to the present invention that is effective when administered alone or in combination. This phrase is also intended to include an amount of a combination of the claimed compounds that is effective to stimulate endogeneous regeneration of terminally differentiated cells in mammals. Preferably, said combination of compounds is a synergistic combination. Such synergy occurs when the effect of the compounds when administered in combination is greater than the additive effect of the compounds when administered alone as a single agent.

The terms "treating" or "treatment", as used herein, cover the treatment of a disorder-state in a mammal, particularly in a human, and include
- Preventing the disorder-state from occurring in a mammal, e.g. said mammal is predisposed to the disorder, but is not diagnosed to have that disorder,
- Inhibiting the disorder-state, i.e. stopping further development, and/or
- Relieving the disorder-state, i.e. improving the symptoms of the disorder.

The claimed compounds and the claimed pharmaceutical composition/medicament may be administered to a mammal in different dosage forms. Preferred is a dosage form allowing direct administration of the compound to the damaged cells or tissues, e.g. into the cochlea of the mammal. Therefore, according to one embodiment of the invention non-oral dosage forms are preferred, in particular as injections. In these cases, administration onto or into the inner ear takes place, for example, transtympanally by injection into the middle ear, by application onto the round or oval window of the inner ear or by (direct) injection into the inner ear. In this context, e.g. pumps or similar devices may be employed.

It is also possible to apply the compounds (pharmaceutical composition, medicament) systemically, e.g. in an oral dosage form. These dosage forms include granules, powders, tablets or capsules, sirups, emulsions, suspensions etc..

All dosage forms can be manufactured by per se known techniques conventionally used in pharmaceutical procedures, for example by mixing, granulation or layering methods. The pharmaceutical compositions or medicaments may additionally be sterilized.

The exact dosage (therapeutically effective amount) of the compounds or the pharmaceutical composition/medicament according to the invention can be selected appropriately according to the recipient, its age and body weight, current clinical status, administration time, dosage form, method of administration, the compound actually employed and, if appropriate, other pharmaceuticals used.

A dose range, preferably an oral dose range, for an adult recipient may be selected between 0,01 to 10 mg/kg body weight, preferably 0,05 to 10 mg/kg body weight, more preferably 0,05 to 5 mg/kg body weight. In the treatment of an inner ear hearing loss after damage or loss of sensory hair cells in an organ of Corti the dosage can be related to the "number of inner ears treated" and/or to the "number of administration". The reason is, that a repeated administration of the compound/pharmaceutical composition over a time period, e.g. between a number of days and a number of weeks/months, preferably at intervals of some days (1 to 7 days), is appropriate. In these cases, the amount of active compound employed, preferably directly to the cochlea as described earlier, e.g. via infusion, should be in the range of from 0,5 µg to 1.0 mg per inner ear and administration.

### Experimental part and examples

### PART 1

In the following a synthetic route for providing the novel compounds according to the invention is shown.

According to this route in scheme 1 substituent R1 of the compound of formula I is provided via compound **3.**

According to scheme 2 compound **3** (corresponding to substituent R1) is incorporated into compound **5.** Further, according to scheme 2 alkyl is introduced as substituent R2 into compound **6.** After cleavage of compound **6** (scheme 2), in a number of synthetic steps according to scheme 3, the structure of the oxazole carboxylic acid (including substituents R1 and R2) is provided (see compound **11** of scheme 3).

According to scheme 4 the corresponding carboxylic acid amide is formed via a reaction with the corresponding amine (providing substituent R3). After removal of the amine protecting group BOC from compound **12** target compound **13** is provided.

Inspite of the fact that the synthetic route is disclosed in a general way for providing a number of the novel compounds according to the present invention, compound **13** is shown as a specific compound, namely as (S)-2-(2-amino-1-(1H-indol-3-yl)propan-2-yl)-N-cyclohexyloxazole-4-carboxamide hydrochloride.

Compound **12** can be modified into compound **12a** according to scheme 5 by substituting hydrogen H at the heteroatom N of the indolyl group, preferably by alkyl (substituent R4).

Referring to formula I compound **12** and compound **13** can also be defined as compounds, wherein
- X is 0 (oxygen),
- R1 is (1 H-indol-3-yl)-methyl, if applicable substituted, in particular at the heteroatom N of the indolyl group, preferably by alkyl, preferably by methyl.
- R2 is alkyl, preferably methyl, and
- R3 is cycloalkyl, preferably cyclohexyl. 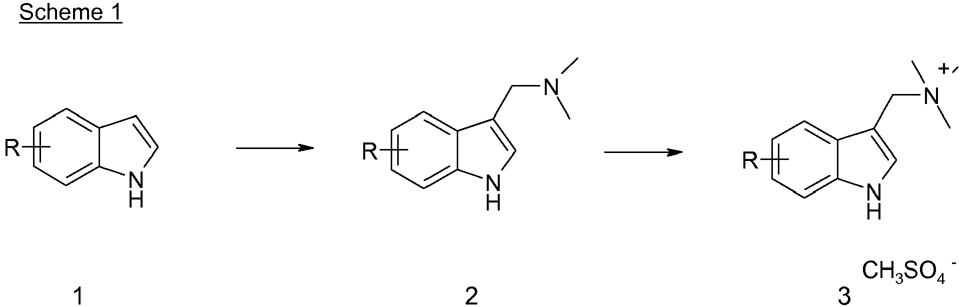 3-Dimethylaminomethylindoles **2:** To a mixture of formalin (37% in water, 0.61mL, 8.14mmol), 1,4-dioxane, and AcOH (7mL) Me₂NH (40% in water, 1.03mL, 8.14mmol) was added at 0 °C. A solution of indole **1** (7.40mmol) in 1,4-dioxane (7mL) was added at 0 °C gradually. After stirring at 0 °C for 2 h and at room temperature for 18 h, water (9mL), charcoal (0.43g), and celite (0.43g) were added and the reaction mixture was filtered through a celite pad. The pH of the filtrate was adjusted to 12 by 2N NaOH in water. Filtration, washing (water), and drying of the precipitate afforded **2.** Yields ca 90%

Indol-3-yl-methyl-trimethylammonium methylsulfate **3:** To a solution of dimethylsulfate (3.94g, 31.2mmol) and AcOH (89 µL, 1.56 mmol) in THF (Tetrahydrofuran) (4mL) a solution of **2** (1.20g, 6.24mmol) and AcOH (89 µL, 1.56 mmol) in THF (10mL) were added dropwise at 10-15 °C. After stirring at 0 °C for 1 h, the precipitates were filtered, washed with Et₂O (Diethyl ether), and subsequently with DCM (Dichloromethane) to afford **3.** Yields: 95-98%.

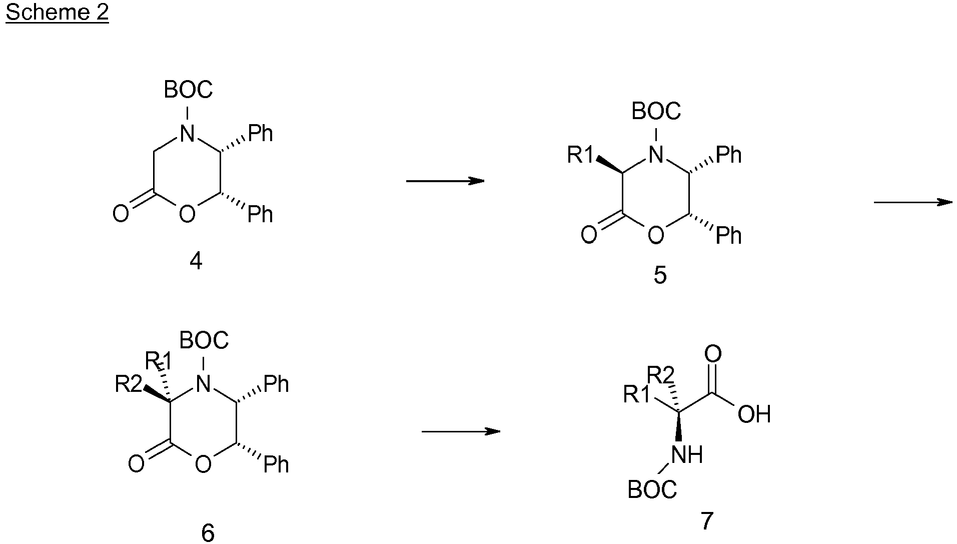

Alkylation of **4:** To a solution of (2S,3R)-(+)-N-Boc-6-oxo-2,3-diphenylmorpholine **4** (500 mg. 1.42 mmol) in THF (30mL) a solution of LDA (Lithium diisopropylamide) (1.0 M in THF, 2.9 mL, 2.97mmol) was added at -78 °C dropwise. Boc or BOC refers to the Amine protecting group tert-Butyloxycarbonyl. After stirring at -78 °C for 30 min, a solution of Li₂CuCl₄ (0.1 M in THF, 1.41mL, 0.141 mmol) was added dropwise followed by a slow addition of a suspension of 3 (1.56mmol) in THF (20mL). After stirring at -78 °C for 1 h, the reaction mixture was allowed to reach room temperature (30 min) and then saturated (sat.) solution of NH₄Cl in water was added. Extractive workup (EtOAc (Ethyl acetate), water, brine) and concentration of the EtOAc extracts followed by column chromatography afforded compound **5,** exemplified by
(3R,5R,6S)-tert-butyl-3-((1H-indol-3-yl)-methyl)-2-oxo-5,6-diphenylmorpholine-4-carboxylate, yield: 75%
(3R,5R,6S)-tert-butyl-3-((7-fluoro-1 H-indol-3-yl)-methyl)-2-oxo-5,6-diphenylmorpholine-4-carboxylate, yield: 71%
(3R,5R,6S)-tert-butyl-3-((7-methyl-1 H-indol-3-yl)-methyl)-2-oxo-5,6-diphenylmorpholine-4-carboxylate, yield: 41%
Alkylation of 5: To a solution of 5 (1.24mmol) in THF (6mL) a solution of LDA (1.0 M in THF, 2.5 mL, 250mmol) was added at -78 °C dropwise. After stirring at -78 °C for 30 min, a solution of alkyliodide (1.36mmol) in THF (2 mL) was added. After stirring at -78 °C for 2 h, the reaction mixture was allowed to reach room temperature (30 min) and then sat. solution of NH₄Cl in water was added. Extractive workup (EtOAc, water, brine) and concentration of the EtOAc extracts followed by column chromatography afforded **6,** exemplified by
(3S,5R,6S)-tert-butyl-3-((1H-indol-3-yl)methyl)-3-methyl-2-oxo-5,6-diphenylmorpholine-4-carboxylate, yield: 62%
Cleavage of chiral auxiliary: Lithium (164mg, 23.7mmol) was added to liquid ammonia (approx. 25 mL) at -78 °C in one portion. After stirring at -78 °C for 30 min, t-BuOH (450µL, 4.74mmol) and a solution of **6** (0.795 mmol) in THF (5mL) were added dropwise. The reaction mixture was stirred at -78 °C for 25 min, then quenched with NH₄Cl (2.26g, 42.3mmol). A cooling bath was removed and ammonia was allowed to vaporize. Extractive workup (EtOAc, 10 % citric acid in water, brine) and concentration of the EtOAc extracts followed by column chromatography afforded **7,** exemplified by
(S)-2-(tert-butoxycarbonylamino)-3-(1H-indol-3-yl)-2-methylpropanoic acid, yield: 86%

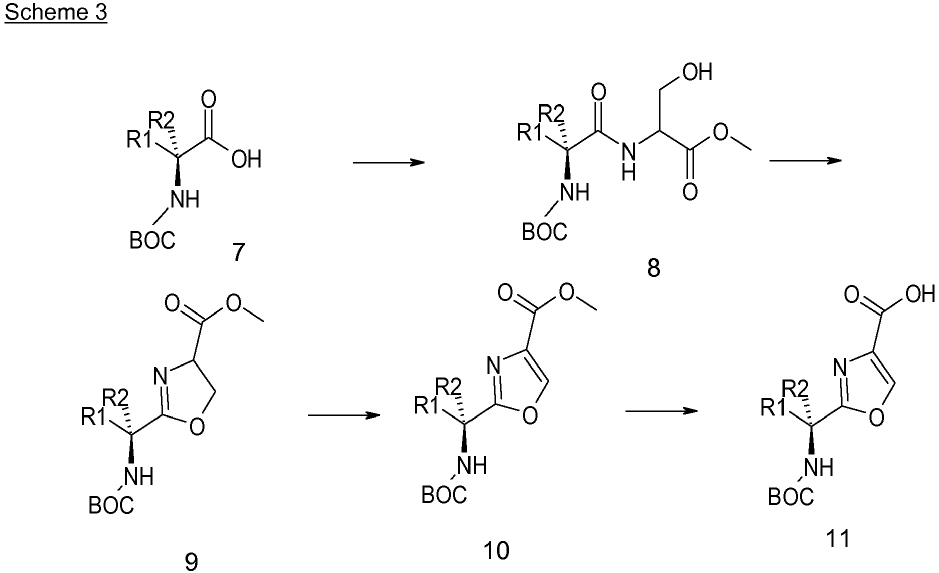

To a solution of **7** (0.29mmol) and (S)-methyl-2-amino-3-hydroxypropanoate hydrochloride (68mg, 0.44mmol) in DMF (Dimethylformamide) (5mL) and DCM (4mL), EDC (1-Ethyl-3-(3-dimethylaminopropyl) carbodiimide) hydrochloride (61 mg, 0.32mmol), HOBt (Hydroxybenzotriazole) (43mg, 0.32mmol), and NMM (N-Methylmorpholine) (112µL, 1.01mmol) were successively added at 0 °C. The reac-tion mixture was stirred at room temperature for 2h. Extractive workup (EtOAc, sat. NaHCO₃ in water, sat. NH₄Cl in water, water, brine) and concentration of the EtOAc extracts followed by column chromatography afforded **8,** exemplified by
(S)-Methyl-2-((S)-2-methyl-2-(tert-butoxycarbonylamino)-3-(1H-indol-3-yl)propanamido)-3-hydroxypropanoate, yield:72%
Synthesis of oxazolines: To a solution of **8** (0.26mmol) in DCM (3mL) DAST (Diethylaminosulfur trifluoride) (38µL, 0.286mmol) was added at -78 °C dropwise. After stirring at -78 °C for 2h, K₂CO₃ (54mg, 0.39mmol) was added in one portion. The reaction mixture was allowed to reach room temperature (2 h) and then stirred at room temperature for 16 h. Extractive workup (DCM, sat. NaHCO₃ in water, brine) and concentration of the DCM extracts afforded compounds **9** that are used in the next step without additional purification.

Synthesis of oxazoles: To a solution of **9** (0.247mmol) in DCM (5mL) DBU (1,8-Diazabicycloundec-7-ene) was added at -30 °C. After stirring at -30 °C for 10 min, to the mixture CBrCl₃ (59µL, 0.60mmol) was added and the mixture was stirred for 18 h allowing gradually warming up to room temperature. Extractive workup (DCM, 10% citric acid, brine) and concentration of the DCM extracts followed by column chromatography afforded **10,** exemplified by
(S)-Methyl-2-(2-(tert-butoxycarbonylamino)-1-(1H-indol-3-yl)-propan-2-yl)oxazole-4-carboxylate, yield: 80%
Hydrolysis of esters **10:** To a solution of **10** (0.14mmol) in THF/MeOH (7/1, 2mL) (MeOH = Methanol) a solution of LiOH (2M in water, 210µL, 0.42 mmol) was added and the reaction mixture was stirred at room temperature for 2 h. pH of the medium was adjusted to 3 by 0.1 HCl in water. Extractive workup (EtOAc, brine) and concentration of the EtOAc extracts afforded **11,** exemplified by
(S)-2-(2-(tert-butoxycarbonylamino)-1-(1H-indol-3-yl)-propan-2-yl)oxazole-4-carboxylic acid, yield: 95%

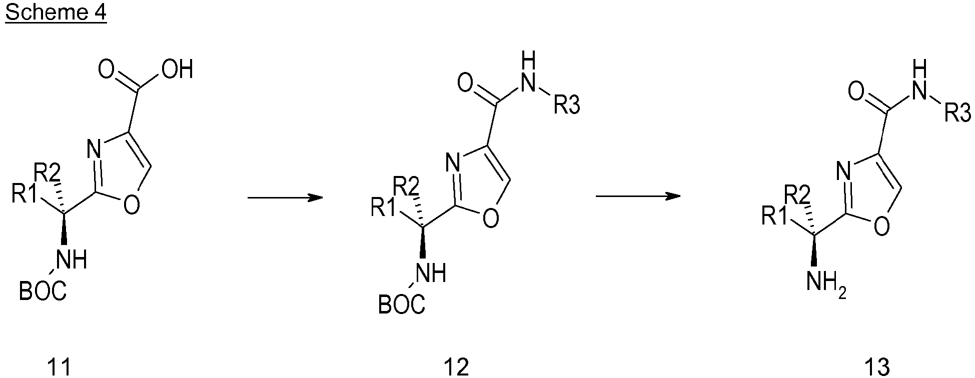

Synthesis of amides **12:** To a solution of **11** (0.13mmol), EDC hydrochloride (30mg. 0.16mmol), and HOBt (21mg, 0.16mmol) in DCM (5mL), NMM (21µL, 0.19mmol) and amine (0.16mmol) were successively added. The reaction mixture was stirred at room temperature for 4h. Extractive workup (DCM, 10 % citric acid in water, sat. NaHCO₃ in water, brine) and concentration of the DCM extracts followed by column chromatography afforded 12, exemplified by
(S)-tert-butyl-2-(4-(cyclohexylcarbamoyl)oxazol-2-yl)-3-(1H-indol-3-yl)propylcarbamate, yield: (68%).

Removal of Boc-group: To a solution of **12** (0.08mol) in DCM (2mL) a solution of HCl (5M in iPrOH, 0.32mL, 1.6mmol) (iPrOH = Isopropyl alcohol) was added and reaction mixture was stirred at 35 °C for 2 h. Evaporation of the solvents and drying of residue in vacuum (0.1 mmHg) afforded the target compound **13,** exemplified by
(S)-2-(2-amino-1-(1H-indol-3-yl)propan-2-yl)-N-cyclohexyloxazole-4-carboxamide hydrochloride, yield: 100%

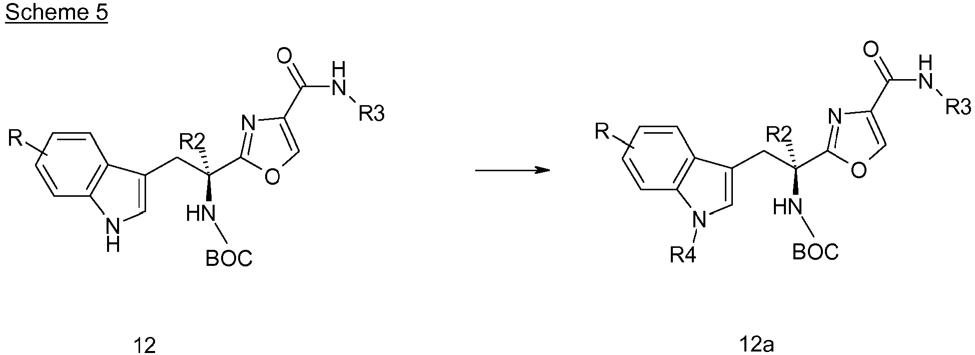

To a solution of **12** (90mg, 0.20mmol) in THF (3mL) at 10 °C t-BuOK (25mg, 0.22mmol) (t-BuOK = Potassium tert-butoxide) was added. After stirring at 15 °C for 45 min, to the mixture alkyliodide (0.40mmol) was added and stirring was continued for 1.5 h at 15 °C. Concentration, extractive workup (EtOAc, water, brine) and concentration of the EtOAc extract followed by column chromatography afforded compound **12a.**

### PART 2

A screening assay was employed according to an experimental protocol that enables the isolation of inner ear progenitor cells from the postnatal sensory organ of the mouse preparations in vitro. These isolated progenitors generate typical spheres that express a comprehensive set of marker genes that define the early developing sensory epithelium in the progenitor cell state. Under appropriate conditions these spheres can differentiate into epithelial patches that express marker genes that are characteristic of differentiating hair cells and supporting cells as found in the native organ of Corti. These differentiated sensory epithelial patches, tagged "mini ears", can be generated in sufficient numbers for screening purposes in a low-throughput format. For an appropriate read-out, dedifferentiation was detected with three parameters: (i) labelling of Sox2 expression (Sox2 = transcription factor SRY (sex determining region Y-box 2), (ii) an increase in the incorporation of EdU indication proliferation (EdU = thymidine analogue 5-Ethynyl-2'-deoxyuridine), and (iii) labelling using the hair cell marker Myosin VIIA.

Compounds were screened at a standard concentration of 5 µM. Initial solution of 5 mM was prepared in DMSO (Dimethylsulfoxide), and aliquots of the stock solution were stored at -20°C. For assay reference 0.1 % DMSO was used.

Test-items were tested in three independent experiments with a set of three to four wells per compound in each experiment for a total of 9-12 wells for each compound. The inner ear progenitor cells were isolated from the sensory organ of postnatal mice at postnatal day 0. The sensory organ was dissociated by enzymatic and mechanical dissociation to obtain a single cell suspension. The single cells were then plated to form spheres for 5 days in suspension cell culture environment, and in presence of growth factors. After 5 days of proliferation, the spheres in the cell culture suspension were plated on Matrigel coated wells for 14 days of differentiation, in growth factors depleted medium. The spheres in the cell culture suspension attached to the cell culture flask and formed patches. 24 h after plating, the patches were treated with a Notch inhibitor for 24h. The medium was then renewed every 4 days (day 6, 10). After 14 days in differentiation, the cell cultures (patches) were treated with the test item for 96 h at a final concentration of 5 µM.

For the last 5 hours of the treatment the patches were exposed to EdU (Click-it EdU Cell Proliferation Assay, Life Invitrogen) to evaluate the proliferative potential of each test item. The staining for EdU was performed according to the manufacturer. In addition a hair cell marker (i.e. Myosin VIIa) and a supporting cell marker (i.e. Sox2) were stained, a nuclear counterstain (i.e. DAPI (4,6-diamidino-2-phenylindole) was used.

For ease of comparison the number of EdU positive cells was normalized to the DMSO only condition. Thus, the proliferation activity is expressed as "fold EdU positive cells". Using proliferation medium as a positive control, an increase proliferation activity by 2.1 fold compared to DMSO was found. Unaltered proliferation activity was found using the differentiation medium. The exemplary compound 13 (see examples, part 1), an increase proliferation activity by 2 fold compared to DMSO.

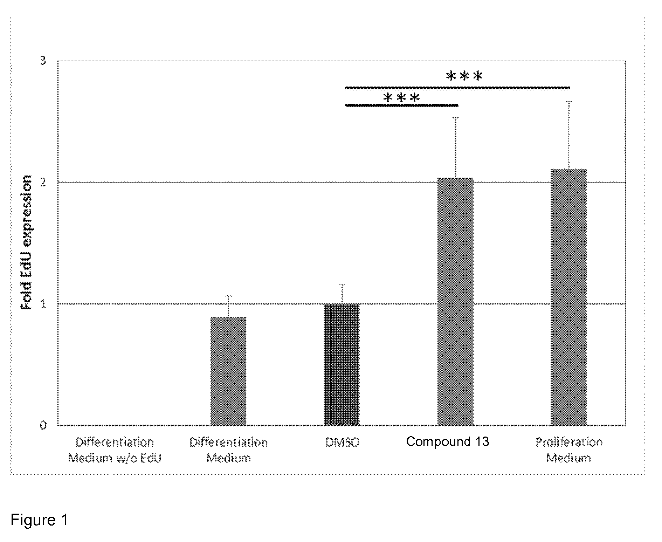

The corresponding results are shown in Figure 1 and Tables 1 and 2.

Figure 1 shows the proliferation activity expressed as "fold EdU positive cells". The positive control measured proliferation medium showed about 2 fold proliferation activity, the differentiation medium showed activity similar to DMSO. These results fully meet the anticipated behavior of the assay. The exemplary compound 13 showed similar proliferation inducing activity as the positive control. *** indicates (statistical) significance p< 0.0001.

Table 1 shows a summary of results: Test-Items, number of differentiated spheres analyzed, mean per-centage of EdU positive cells, Standard error of the Mean (SEM), Proliferation activity expressed as "fold EdU positive cells".

**Table 1**

| Condition (test item) | Number of differentiated spheres | Mean percentage of EdU positive cells | SEM | Fold EdU positive re DMSO |
|---|---|---|---|---|
| Diff. Medium | 639 | 1.57 | 0.18 | 0.89 |
| DMSO | 683 | 1.76 | 0.16 | 1.00 |
| Compound 13 | 243 | 3.59 | 0.49 | 2.04 |
| Prolif. Medium | 224 | 3.71 | 0.56 | 2.11 |

Table 2 shows a statistical (T-Test) comparison of the Test-items to reference conditions.

**Table 2**

| Compound | Test-Item | p-Value |
|---|---|---|
| 13 | Diff. Medium | <.0001 |
| 13 | Prolif. Medium | 0.8006 |
| 13 | DMSO | <.0001 |

## Claims

1. A compound of formula I 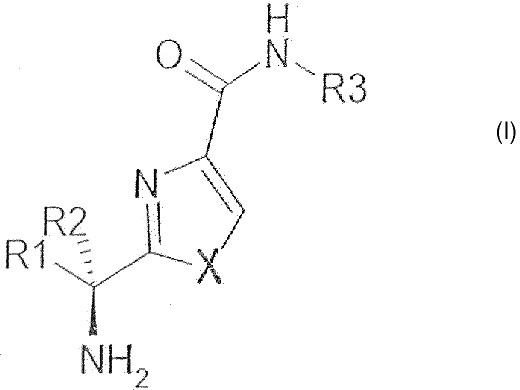 wherein
- X is O (oxygen) or S (sulphur),
- R1 is a substituent selected from the group consisting of straight-chain (unbranched) or branched, unsubstituted or substituted aryl groups, alkylaryl groups, and arylalkyl groups which optionally contain heteroatoms,
- R2 is a substituent selected from the group consisting of straight-chain (unbranched) or branched, unsubstituted or substituted C1 - C6 alkyl groups, C1 - C6 alkoxy groups, C1 - C6 alkoxy alkyl groups and C2 - C6 alkenyl groups,
- R3 is is a substituent selected from the group consisting of straight-chain (unbranched) or branched, unsubstituted or substituted alkyl groups, cycloalkyl groups, alkylcycloalkyl groups, aryl groups, alkylaryl groups, arylalkyl groups, cycloalkylaryl groups and arylcycloalkyl groups, which optionally contain heteroatoms,
- or a stereoisomer, a tautomer or a pharmaceutically acceptable salt thereof.

2. The compound according to claim 1, wherein X is O (oxygen).

3. The compound according to claim 1 or claim 2, wherein R1 is a substituent selected from the group consisting of straight-chain or branched, unsubstituted or substituted heteroaryl groups, alkylheteroaryl groups, and heteroarylalkyl groups, in particular indolyl groups, alkylindolyl groups, and indolylalkyl groups.

4. The compound according to anyone of the preceding claims, wherein R2 is a substituent selected from the group consisting of straight-chain or branched, unsubstituted or substituted C1 - C6 alkyl groups, in particular C1 - C3 alkyl groups, especially methyl groups.

5. The compound according to anyone of the preceding claims, wherein R3 is a substituent selected from the group consisting of straight-chain or branched, unsubstituted or substituted alkyl groups, cycloalkyl groups, and alkylcycloalkyl groups.

6. The compound according to claim 5, wherein R3 is a substituent selected from the group consisting of unsubstituted or substituted cycloalkyl groups.

7. The compound according to anyone of the preceding claims, wherein
- X is O (oxygen),
- R1 is a substituent selected from the group consisting of straight-chain or branched, un-substituted or substituted indolyl groups, alkylindolyl groups and indolylalkyl groups,
- R2 is a substituent selected from the group consisting of straight-chain or branched, un-substituted or substituted C1 - C3 alkyl, in particular methyl,
- R3 is a substituent selected from the group consisting of unsubstituted or substituted cycloalkyl groups, in particular cyclohexyl groups.

8. The compound according to claim 7, wherein
- X is O (oxygen),
- R1 is (1 H-indol-3-yl)-methyl,
- R2 is methyl,
- R3 is cyclohexyl.

9. The compound according to anyone of the preceding claims for use as a pharmaceutical composition or a medicament.

10. A pharmaceutical composition or medicament, comprising:
- at least one compound according to anyone of claims 1 to 8, and
- a pharmaceutically acceptable carrier or diluent.

## Patentansprüche

1. Verbindung mit der Formel I 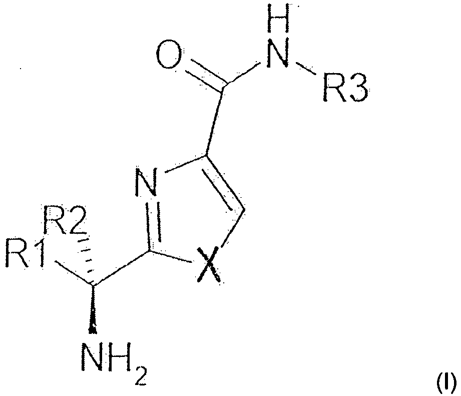 worin
- X ein O (Sauerstoffatom) oder ein S (Schwefelatom) darstellt,
- R1 einen Substituenten darstellt, der ausgewählt ist aus der Gruppe bestehend aus geradkettigen (unverzweigten) oder verzweigten, unsubstituierten oder substituierten Arylgruppen, Alkylarylgruppen und Arylalkylgruppen, die optional Heteroatome enthalten,
- R2 einen Substituenten darstellt, der ausgewählt ist aus der Gruppe bestehend aus geradkettigen (unverzweigten) oder verzweigten, unsubstituierten oder substituierten C1-C6-Alkylgruppen, C1-C6-Alkoxygruppen, C1-C6-Alkoxyalkylgruppen und C2-C6-Alkenylgruppen,
- R3 einen Substituenten darstellt, der ausgewählt ist aus der Gruppe bestehend aus geradkettigen (unverzweigten) oder verzweigten, unsubstituierten oder substituierten Alkylgruppen, Cycloalkylgruppen, Alkylcycloalkylgruppen, Arylgruppen, Alkylarylgruppen, Arylalkylgruppen, Cycloalkylarylgruppen und Arylcycloalkylgruppen, die optional Heteroatome enthalten,
- oder ein Stereoisomer, ein Tautomer oder ein pharmazeutisch akzeptables Salz davon.

2. Verbindung nach Anspruch 1, worin X ein O (Sauerstoffatom) darstellt.

3. Verbindung nach Anspruch 1 oder Anspruch 2, worin R1 einen Substituenten darstellt, der ausgewählt ist aus der Gruppe bestehend aus geradkettigen oder verzweigten, unsubstituierten oder substituierten Heteroarylgruppen, Alkylheteroarylgruppen und Heteroarylalkylgruppen, insbesondere Indolylgruppen, Alkylindolylgruppen und Indolylalkylgruppen.

4. Verbindung nach einem der vorhergehenden Ansprüche, worin R2 einen Substituenten darstellt, der ausgewählt ist aus der Gruppe bestehend aus geradkettigen oder verzweigten, unsubstituierten oder substituierten C1-C6-Alkylgruppen, insbesondere C1-C3-Alkylgruppen, speziell Methylgruppen.

5. Verbindung nach einem der vorhergehenden Ansprüche, worin R3 einen Substituenten darstellt, der ausgewählt ist aus der Gruppe bestehend aus geradkettigen oder verzweigten, unsubstituierten oder substituierten Alkylgruppen, Cycloalkylgruppen und Alkylcycloalkylgruppen.

6. Verbindung nach Anspruch 5, worin R3 einen Substituenten darstellt, der ausgewählt ist aus der Gruppe bestehend aus unsubstituierten oder substituierten Cycloalkylgruppen.

7. Verbindung nach einem der vorhergehenden Ansprüche, worin
- X ein O (Sauerstoffatom) darstellt,
- R1 einen Substituenten darstellt, der ausgewählt ist aus der Gruppe bestehend aus geradkettigen oder verzweigten, unsubstituierten oder substituierten Indolylgruppen, Alkylindolylgruppen und Indolylalkylgruppen,
- R2 einen Substituenten darstellt, der ausgewählt ist aus der Gruppe bestehend aus geradkettigem oder verzweigtem, unsubstituiertem oder substituiertem C1-C3-Alkyl, insbesondere Methyl,
- R3 einen Substituenten darstellt, der ausgewählt ist aus der Gruppe bestehend aus unsubstituierten oder substituierten Cycloalkylgruppen, insbesondere Cyclohexylgruppen.

8. Verbindung nach Anspruch 7, worin
- X ein O (Sauerstoffatom) darstellt,
- R1 ein (1H-Indol-3-yl)-methyl darstellt,
- R2 ein Methyl darstellt,
- R3 ein Cyclohexyl darstellt.

9. Verbindung nach einem der vorhergehenden Ansprüche zur Verwendung als pharmazeutische Zusammensetzung oder als Medikament.

10. Pharmazeutische Zusammensetzung oder Medikament, umfassend:
- mindestens eine Verbindung nach einem der Ansprüche 1 bis 8 und
- einen pharmazeutisch akzeptablen Träger oder ein pharmazeutisch akzeptables Verdünnungsmittel.

## Revendications

1. Composé de formule I 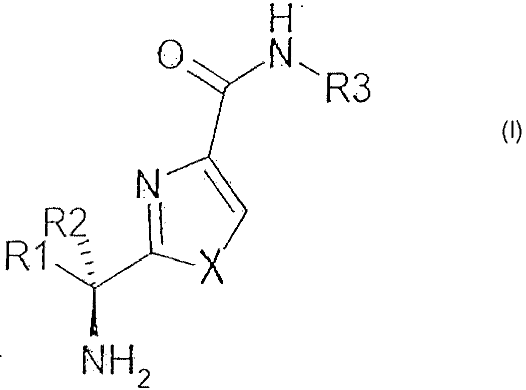 où
- X représente 0 (oxygène) ou S (soufre),
- R1 représente un substituant choisi dans le groupe constitué par les groupements aryle, arylalkyle ou alkylaryle linéaires (non ramifiés) ou ramifiés, non substitués ou substitués, qui comportent éventuellement des hétéroatomes,
- R2 représente un substituant choisi dans le groupe constitué par les groupements alkyle en C1 - C6, alkoxy en C1 - C6, alkoxyalkyle en C1 - C6 et alcényle en C2 - C6 linéaires (non ramifiés) ou ramifiés, non substitués ou substitués, R3 représente un substituant choisi dans le groupe constitué par les groupements alkyle, cycloalkyle, alkylcycloalkyle, aryle, alkylaryle, arylalkyle, cycloalkylaryle et arylcycloalkyle linéaires (non ramifiés) ou ramifiés, non substitués ou substitués, qui comportent éventuellement des hétéroatomes,
- - ou stéréoisomère, tautomère ou sel pharmaceutiquement acceptable de celui-ci.

2. Composé selon la revendication 1, où X représente 0 (oxygène).

3. Composé selon la revendication 1 ou la revendication 2, où R1 représente un substituant choisi dans le groupe constitué par les groupements hétéroaryle, alkylhétéroaryle et hétéroarylalkyle linéaires ou ramifiés, non substitués ou substitués, en particulier les groupements indolyle, alkylindolyle et indolylalkyle.

4. Composé selon l'une quelconque des revendications précédentes, où R2 représente un substituant choisi dans le groupe constitué par les groupements alkyle en C1 - C6 linéaires ou ramifiés, non substitués ou substitués, en particulier les groupements alkyle en C1 - C3, spécialement les groupements méthyle.

5. Composé selon l'une quelconque des revendications précédentes, où R3 représente un substituant choisi dans le groupe constitué par les groupements alkyle, cycloalkyle et alkylcycloalkyle linéaires ou ramifiés, non substitués ou substitués.

6. Composé selon la revendication 5, où R3 représente un substituant choisi dans le groupe constitué par les groupements cycloalkyle non substitués ou substitués.

7. Composé selon l'une quelconque des revendications précédentes, où
- X représente O (oxygène),
- R1 représente un substituant choisi dans le groupe constitué par les groupements indolyle, alkylindolyle et indolylalkyle linéaires ou ramifiés, non substitués ou substitués,
- R2 représente un substituant choisi dans le groupe constitué par les groupements alkyle en Cl - C3 linéaires ou ramifiés, non substitués ou substitués, en particulier méthyle,
- R3 représente un substituant choisi dans le groupe constitué par les groupements cycloalkyle non substitués ou substitués, en particulier les groupements cyclohexyle.

8. Composé selon la revendication 7, où
- X représente O (oxygène),
- R1 représente un groupement (1H-indol-3-yl)-méthyle,
- R2 représente un groupement méthyle,
- R3 représente un groupement cyclohexyle.

9. Composé selon l'une quelconque des revendications précédentes, pour utilisation comme composition pharmaceutique ou médicament.

10. Composition pharmaceutique ou médicament, comprenant :
- au moins un composé selon l'une quelconque des revendications 1 à 8, et
- un vecteur ou diluant pharmaceutiquement acceptable.
